# EUROPÄISCHE PATENTSCHRIFT

(11) **EP 1 200 048 B1**
(45) Veröffentlichungstag und Bekanntmachung des Hinweises auf die Patenterteilung: **07.03.2007**
(21) Anmeldenummer: 00949398.2
(22) Anmeldetag: 22.07.2000
(51) Int. Cl.: A61Q 5/10, A61K 8/49

(54) **NEUE ENTWICKLER-KUPPLER-KOMBINATIONEN**
NOVEL DEVELOPER-COUPLER COMBINATIONS
NOUVELLES ASSOCIATIONS GENERATEUR/COPULANT

(30) Priorität: 03.08.1999 DE 19936442
(43) Veröffentlichungstag der Anmeldung: 02.05.2002
(60) Teilanmeldung: 07001943.5
(73) Patentinhaber: Henkel Kommanditgesellschaft auf Aktien, 40589 Düsseldorf (DE)
(72) Erfinder: HÖFFKES, Horst, D-40595 Düsseldorf (DE); ROSE, David, D-40723 Hilden (DE)
(86) Internationale Anmeldenummer: PCT/EP2000/007052
(87) Internationale Veröffentlichungsnummer: WO 2001/008645

(56) Entgegenhaltungen:
- EP-A- 0 398 702
- WO-A-00/61087
- WO-A-00/61089
- WO-A-00/61090
- WO-A-92/19220
- WO-A-99/11228
- FR-A- 2 751 218
- FR-A- 2 769 211

## Beschreibung

Die vorliegende Erfindung betrifft Mittel zum Färben keratinischer Fasern, insbesondere menschlicher Haare, die Kombinationen spezieller Oxidationsfarbstoffvorprodukte enthalten, sowie deren Verwendung.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehrerer Kupplerkomponenten die eigentlichen Farbstoffe aus.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt. Als Kupplerkomponenten werden in der Regel m-Phenylendiaminderivate, Naphthole, Resorcin und Resorcinderivate, Pyrazolone und m-Aminophenole verwendet.

Allein mit einer Entwicklerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt. Es besteht daher ständig Bedarf an neuen, verbesserten Farbstoffkombinationen. Die durch die Farbstofflcombinationen erzielbaren Färbungen sollen beständig sein gegen Licht, Wärme, Schweiß, Reibung und den Einfluß chemischer Reduktionsmittel, z.B. Dauerwellenflüssigkeiten. Weiterhin soll die erzielte Färbung durch Blondierung leicht wieder aus dem Haar entfernt werden können, falls sie doch nicht den individuellen Wünschen der einzelnen Person entspricht und rückgängig gemacht werden soll. Daher bestand nach wie vor die Aufgabe, neue Entwickler-Kupplerkombinationen zu entwickeln, mit denen sich ausdrucksvolle, brillante Farbtöne, insbesondere im Rot/Rotbraun-Bereich, erzielen lassen und die auch in toxikologischer Hinsicht ein Fortschritt gegenüber dem Stand der Technik sind.

In der FR-2 769 211 werden enzymatische Haarfärbemittel offenbart, die ein p-Aminophenolderivat in Kombination mit m-Aminophenolderivaten enthalten.
In der WO-00/61090, WO-00/61087 und WO-00/61089 werden jeweils oxidative Haarfärbemittel beschrieben, die 4-Amino-2-((diethylamino)-methyl)-phenol als Entwicklerkomponente enthalten.
Ferner werden in der WO99/11228 oxidative Haarfärbemittel offenbart, die p-Aminophenole in Kombination mit m-Aminophenolen enthalten.

Es wurde nun überraschenderweise gefunden, daß bestimmte Farbstoffkombinationen die an Oxidationshaarfarben gestellten Aufgaben in einem hohen Maße erfüllen.

Ein erster Gegenstand der vorliegenden Erfindung sind daher Mittel zum Färben von Keratinfasern, insbesondere menschlichen Haaren, die in einem zum Färben geeigneten Medium mindestens ein p-Aminophenolderivat und mindestens ein Oxidationsmittel, ausgewählt aus Persulfaten, Chloriten sowie Wasserstoffperoxid oder dessen Anlagerungsprodukten an Harnstoff, Melamin sowie Natriumcarbonat, enthalten, und weiterhin mindestens eine Verbindung der Formel (II) oder eines deren physiologisch verträglichen Salze als Kuppierkomponente 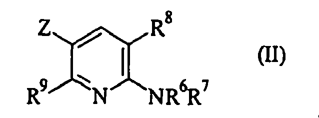 wobei R⁶ und R⁷ unabhängig voneinander stehen für Wasserstoff oder eine C₁₋₄-Alkyl-Gruppe,
R⁸ und R⁹ stehen unabhängig voneinander für Wasserstoff, eine Aminogruppe, eine Hydroxygruppe oder eine C₁₋₄-Alkoxygruppe, und
Z steht für Wasserstoff oder ein Halogenatom, insbesondere ein Chloratom, wobei mindestens einer der Reste R⁶, R⁷, R⁹ oder Z von Wasserstoff verschieden ist, enthält, mit der Maßgabe, dass das Mittel frei ist von 4-Amino-2-((diethylamino)-methyl)-phenol, wenn die Verbindung der Formel (II) 3-Amino-2-methylamino-6-methoxypyridin ist.

Unter Keratinfasern sind dabei Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Besonders bevorzugt im Sinne der vorliegenden Erfindung sind Oxidationsfärbemittel in einem wäßrigen Träger oder in Pulverform.

Beispiele für die als Substituenten in den erfindungsgemäßen Verbindungen genannten, C₁- bis C₄-Alkylgruppen sind die Gruppen Methyl, Ethyl, Propyl, Isopropyl und Butyl. Ethyl und Methyl sind bevorzugte Alkylgruppen. Eine erfindungsgemäß bevorzugte C₁- bis C₄-Alkoxygruppe, ist beispielsweise eine Methoxy- oder eine Ethoxygruppe. Diese Definition der Alkyl- und Alkoxygruppen ist auch anzuwenden, wenn diese Bestandteile eines komplexeren Substituenten sind. So sind bevorzugte Vertreter für eine C₁- bis C₄-Hydroxyalkylgruppe beispielsweise eine Hydroxymethyl-, eine 2-Hydroxyethyl-, eine 3-Hydroxypropyl- oder eine 4-Hydroxybutylgmppe. Beispiele für ein Halogenatom sind erfindungsgemäß ein F-, ein Cl- oder ein Br-Atom, ein Cl-Atom ist ganz besonders bevorzugt.

Obwohl prinzipiell alle Verbindungen der Formel (II) erfindungsgemäß geeignet sind, sind die Pyridinderivate 3-Amino-2-methylamino-6-methoxypyridin, 2,3-Diamino-6-methoxypyridin, 2,6-Diaminopyridin und 2-Amino-3-hydroxy-5-chlorpyridin besonders bevorzugt.

Die Mittel gemäß der vorliegenden Erfindung enthalten neben dem p-Aminophenol-Derivat eine Kupplerkomponente der Formel (II) . Es kann aber auch erfindungsgemäß bevorzugt sein, Färbemittel zu formulieren, in der mindestens 2 Verbindungen der Formeln (I) und (II) enthalten sind. So kann es beispielsweise erfindungsgemäß besonders bevorzugt sein, jeweils eine Verbindung der Formel (I) und eine Verbindung der Formel (II) in den Haarfärbemitteln einzusetzen.

In dieser Ausführungsform enthalten die erfindungsgemäßen Färbemittel mindestens ein Verbindung der Formel (I) oder eines deren physiologisch verträglichen Salze als Kupplerkomponente 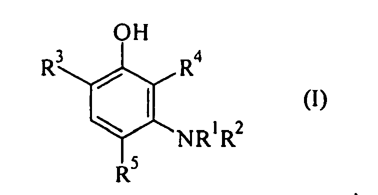 wobei R¹ und R² unabhängig voneinander stehen für Wasserstoff, eine Trifluoracetyl-Gruppe, eine C₁₋₄-Alkylgruppe, eine C₁₋₄-Monohydroxyalkylgruppe, eine C₂₋₄-Polyhydroxyalkylgruppe oder eine C₁₋₄-Aminoalkylgruppe,
R³, R⁴ und R⁵ stehen unabhängig voneinander für Wasserstoff, eine C₁₋₄-Alkyl-Gruppe, eine C₁₋₄-Alkoxy-Gruppe oder ein Halogenatom, insbesondere ein Chloratom, wobei mindestens einer der Reste R³, R⁴ und R⁵ für ein Halogenatom oder eine C₁₋₄-Alkoxy-Gruppe steht.

Obwohl prinzipiell alle Verbindungen der Formel (I) erfindungsgemäß eingesetzt werden können, sind die m-Aminophenolderivate 5-Amino-4-chlor-2-methylphenol, 5-Amino-4-methoxy-2-methylphenol, 3-Trifluoroacetylamino-2-chlor-6-methylphenol, 3-Amino-2-chlor-6-methylphenol, 3-Amino-2,4-dichlorphenol, 3-Amino-6-chlorphenol, 3-Amino-6-bromphenol, 3-(β-Hydroxyethyl)-amino-6-chlorphenol und 3-(β-Aminoethyl)-amino-6-chlorphenol besonders bevorzugt. Ganz besonders bevorzugt sind 5-Amino-4-chlor-2-methylphenol, 3-Trifluoroacetylamino-2-chlor-6-methylphenol, 3-Amino-2-chlor-6-methylphenol und 3-Amino-2,4-dichlorphenol.

Erfindungsgemäß bevorzugte p-Aminophenol-Derivate sind ausgewählt aus Verbindungen der Formel (III) 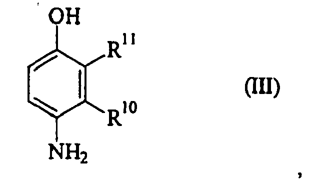 wobei
R¹⁰ und R¹¹ unabhängig voneinander stehen für Wasserstoff, eine C₁₋₄-Alkyl-Gruppe, eine C₁₋₄-Monohydroxyalleyl-Gruppe, eine C₂₋₄-Polyhydroxyalkyl-Gruppe, eine C₁₋₄-Alkoxy-C₁₋₄-Alkyl-Gruppe, eine C₁₋₄-Aminoalkyl-Gruppe, eine (C₁₋₄-Hydroxyalkyl)amino-Gruppe, eine ((C₁₋₄-Hydroxyalkyl)amino)-C₁₋₄-Alkyl-Gruppe, eine ((Di-C₁₋₄-Alkyl)-amino)-C₁₋₄-Alkyl-Gruppe, eine C₁₋₄-Hydroxyalkoxy-Gruppe, eine (2-Hydroxy-5-aminophenyl)-C₁₋₄-alkyl-Gruppe, eine Carboxy-Gruppe oder ein Halogenatom.

Die in dieser Definition verwendeten Begriffe sind analog zu den vorherigen Ausführungen definiert.

Obwohl prinzipiell alle p-Aminophenolderivate der Formel (III) im Sinne der Erfindung bevorzugt sind, sind p-Aminophenol und dessen Derivate Bis-(5-amino-2-hydroxyphenyl)-methan, 2-Aminomethyl-4-aminophenol, 2-(β-Hydroxyethylaminomethyl)-4-aminophenol, 3-Methyl-4-aminophenol, 5-Aminosalicylsäure, 2-Hydroxymethylamino-4-amino-phenol, 2-Hydroxymethyl-4-aminophenol, 4-Amino-2-((diethylamino)-methyl)-phenol, 4-Amino-3-fluorphenol, 4-Amino-2-chlorphenol und 4-Amino-2-(2-hydroxyethoxy)-phenol besonders geeignet. Ganz besonders geeignet sind Bis-(5-amino-2-hydroxyphenyl)-methan, 2-Aminomethyl-4-aminophenol, 4-Amino-2-((diethylamino)-methyl)-phenol, 2-(β-Hydroxyethylaminomethyl)-4-aminophenol, 3-Methyl-4-aminophenol, 4-Amino-2-chlorphenol und 5-Aminosalicylsäure.

Zur Erzielung natürlicher Nuancen ist es häufig notwendig, auch Farbstoffkombinationen einzusetzen, die der Färbung eine gewissen Blau- bzw. Anthrazitanteil geben. Hierzu können prinzipiell sowohl geeignete Entwickler- als auch geeignete Kupplerkomponenten eingesetzt werden. Zu diesem Zweck bevorzugt eingesetzte Entwicklerkomponenten sind p-Phenylendiamin, p-Toluylendiamin, N,N-Bis-(2-hydroxyethyl)-p-phenylendiamin, 1-(2'-Hydroxyethyl)-2,5-diaminobenzol, Bis-(4-aminophenyl)-amin, 1,10-Bis-(2,5-diaminophenyl)-1,4,7,10-tetraoxadecan, 2,6-Dichlor-4-aminophenol, 2,5-Diaminopyridin und 2-Dimethylamino-5-aminopyridin. Als bevorzugte Kupplerkomponenten werden 3,5-Diamino-2,6-dimethoxypyridin, 1-Naphthol, 1,5-Dihydroxynaphthalin, 1,8-Dihydroxynaphthalin, 2,7-Dihydroxynaphthalin, m-Diaminobenzol, 2,4-Diaminophenoxyethanol, 1-Methoxy-2-amino-4-(2'-hydroxyethylamino)-benzol, 1-Amino-3-bis-(2'-hydroxyethyl)-aminobenzol und 1,3-Bis-(2,4-diaminophenoxy)-propan, 1,3-Bis-(2,4-diaminophenyl)-propan, 2,6-Bis-(2-hydroxyethylamino)-1-methylbenzol, 1-Hydroxy-3,4-methylendioxybenzol, 1-Amino-3,4-methylendioxybenzol und 1-(2'-Hydroxyethyl)-amino-3,4-methylendioxybenzol zur Erzielung der genannten Nuancen eingesetzt.

Neben der erfindungsgemäßen Kombination von Oxidationsfarbstoffvorprodukten können die Mittel zur weiteren Nuancierung weitere Oxidationsfarbstoffvorprodukte vom Kuppler- und Entwicklertyp enthalten.

Erfindungsgemäß bevorzugte weitere Entwicklerkomponenten sind o-Aminophenol, 2-(2,5-Diaminophenoxy)-ethanol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 1,4-Bis-(4-aminophenyl)-diazacycloheptan, 1,3-Bis-(N(2-hydroxyethyl)-N-(4-aminophenylami-no))-2-propanol, 1,10-Bis-(2,5-diaminophenyl)-1,4,7,10-tetraoxadecan sowie 4,5-Diamino-pyrazol-Derivate nach EP 0 740 931 bzw. WO 94/08970 wie z.B. 4,5-Diamino-1-(2'-hydroxyethyl)-pyrazol.

Besonders bevorzugte weitere Entwicklerkomponenten sind 2,4,5,6-Tetraaminopyrimidin, 2-Hydroxy-4,5,6-triaminopyrimidin, 4-Hydroxy-2,5,6-triaminopyrimidin, 4,5-Diamino-1-(2-hydroxyethyl)-pyrazol und o-Aminophenol.

Erfindungsgemäß bevorzugte weitere Kupplerkomponenten sind
- m-Aminophenol und dessen Derivate wie beispielsweise 5-Amino-2-methylphenol, 5-(3-Hydroxypropylamino)-2-methylphenol, 2-Hydroxy-4-aminophenoxyethanol, 2,6-Dimethyl-3-aminophenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol und 3-(Ethylamino)-4-methylphenol,
- o-Aminophenol und dessen Derivate,
- m-Diaminobenzol-Derivate,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2,6-Dihydroxy-3,4-dimethylpyridin und 2,6-Dihydroxy-4-methylpyridin,
- Naphthalinderivate wie beispielsweise 2-Methyl-1-naphthol; 2-Hydroxymethyl-1-naphthol, 2-Hydroxyethyl-1-naphthol, 1,6-Dihydroxynaphthalin, 1,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyräzol-5-on,
- Indolderivate wie beispielsweise 4-Hydroxyindol, 6-Hydroxyindol und 7-Hydroxyindol sowie
- Methylendioxybenzolderivate.

Erfindungsgemäß bevorzugte weitere Kupplerkomponenten sind 1,7-Dihydroxynaphthalin, 2-Amino-3-hydroxypyridin, 5-Amino-2-methylphenol, Resorcin, 4-Chlorresorcin, 2-Methylresorcin, 5-Methylresorcin, 2,5-Dimethylresorcin, 2,6-Dihydroxy-3,4-diaminopyridin, 6-Methyl-1,2,3,4-tetrahydrochinoxalin, m-Aminophenol, o-Aminophenol und 2-Chlorresorcin.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1:0,5 bis 1:3, insbesondere 1:1 bis 1:2, enthalten sein können.

In einer bevorzugten Ausführungsform enthalten die erfindungsgemäßen Haarfärbemittel zur weiteren Modifizierung der Farbnuancen neben den Oxidationsfarbstoffvorprodukten zusätzlich übliche direktziehende Farbstoffe. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere in den erfindungsgemäßen Färbemitteln enthaltene Farbstofikomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Amboindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Es ist nicht erforderlich, daß die Oxidationsfarbstoffvorprodukte oder die direktziehenden Farbstoffe jeweils einheitliche Verbindungen darstellen. Vielmehr können in den erfmdungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe), sowie Kapitel 8, Seiten 264-267; Oxidationsfarbstoffvorprodukte), erschienen als Band 7 der Reihe "Dermatology" (Hrg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Händelsunternehmen für Arzneimittel, Reformwaren und Körperpflegemittel e.V., Mannheim, Bezug genommen.

Zur Herstellung der erfindungsgemäßen Färbemittel können die Oxidationsfarbstoffvorprodukte in einen geeigneten wasserhaltigen Träger eingearbeitet werden. Solche Träger sind zum Zwecke der Haarfärbung z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine. 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Alkylamidoamine, insbesondere Fettsäureamidoamine, wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{®} vertriebenen Methyl-hydroxyalkyldialkoyloxyalkyl-ammoniummethosulfate sowie die unter dem Warenzeichen Dehyquart^{®} vertriebenen Produkte wie Dehyquart^{®} AU-46.

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die erfmdungsgemäßen Haarbehandlungsmittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate;
- polymere Dimethyldiallylammoniumsalze und deren Copolymere mit Acrylsäure sowie Estern und Amiden von Acrylsäure und Methacrylsäure. Die unter den Bezeichnungen Merquat^{®}100 (Poly(dimethyldiallylammoniumchlorid)), Merquat^{®}550 (Dimethyldiallylammoniumchlorid-Acrylamid-Copolymer) und Merquat^{®} 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vinylpyrrolidon-Methoimidazoliniumchlorid-Copolymere, wie sie unter der Bezeichnung Luviquat^{®} angeboten werden;
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquatemäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkemöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin. Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropariol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfdrbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Ein zweiter Gegenstand dieser Erfindung ist die Verwendung der vorgenannten Mittel zum Färben keratinischer Fasern.

Die nachfolgenden Beispiele sollen den Erfindungsgegenstand näher erläutern.

### Ausführungsbeispiele

### 1. Herstellung der Färbecreme

### 1.1 Teilmischung A

| | |
|---|---|
| Hydrenol^{®} D¹ | 8,50g |
| Lorol^{®} techn.² | 2,00g |
| Eumulgin^{®} B2³ | 0,75g |
| Texapon^{®} NSO⁴ | 20,00g |
| Dehyton^{®} K⁵ | 12,50g |
| Wasser | 30,00g |

| | |
|---|---|
| ¹ C₁₆₋₁₈-Fettalkohol (INCI-Bezeichnung: Cetearyl alcohol) (HENKEL) ² C₁₂₋₁₈-Fettalkohol (INCI-Bezeichnung: Coconut alcohol) (HENKEL) ³ Cetylstearylalkohol mit ca. 20 EO-Einheiten (INCI-Bezeichnung: Ceteareth-20) (HENKEL) ⁴ Laurylethersulfat, Natriumsalz (ca. 27,5% Aktivsubstanz; INCI-Bezeichnung: Sodium Laureth Sulfate) (HENKEL) ⁵ N,N-Dimethyl-N-(C₈₋₁₈-kokosamidopropyl)ammoniumacetobetain (ca. 30% Aktivsubstanz; INCI-Bezeichnung: Aqua (Water), Cocamidopropyl Betaine) (HENKEL) | |

Die Substanzen Hydrenol^{®} D, Lorol^{®} und Eumulgin^{®} B2 wurden bei 80°C aufgeschmolzen, mit dem 80°C heißen Wasser, enthaltend Texapon^{®} NSO und Dehyton^{®} K, vermischt und unter starkem Rühren emulgiert. Danach wurde die Emulsion unter schwachem Rühren abgekühlt.

### 1.2 Teilmischung B

| | |
|---|---|
| Natriumsulfit | 1,00g |
| Ammoniumsulfat | 1,00g |
| Farbstoffvorprodukte | jeweils 2,5mmol |
| Ammoniak (25%ige Lösung) | ad pH=10,0 |
| Wasser | 10,00g |

Die Farbstoffvorprodukte wurden in dem 50°C heißem Wasser unter Zugabe von Natriumsulfit, Ammoniumsulfat und Ammoniak gelöst.
Die Farbstoffvorproduktlösung (Teilmischung B) wurde zur Emulsion (Teilmischung A) gegeben, mit Ammoniak auf pH=10 eingestellt und mit Wasser auf 100g aufgefüllt. Es wurde bis zum Erreichen der Raumtemperatur weitergerührt.

### 2. Färbung der keratinischen Fasern

Die so erhaltene Färbecreme wurde im Verhältnis 1:1 mit Wasser, einer 1%-igen H₂O₂-Lösung beziehungsweise einer 9%-igen H₂O₂-Lösung vermischt und auf Strähnen standardisierten, zu 80% ergrauten, aber nicht besonders vorbehandelten Menschenhaares (Fa. Klugmann) aufgetragen. Nach 30min Einwirkzeit bei 32°C wurde das Haar gespült, mit einem üblichen Haarwaschmittel ausgewaschen und anschließend getrocknet.

Die Ergebnisse der Färbeversuche sind der folgenden Tabellen zu entnehmen, in der die folgenden Bezeichnungen verwendet wurden:
- E1: 2-Aminomethyl-4-aminophenol
- E2: Bis-(5-amino-2-hydroxyphenyl)-methan
- E3: 3-Methyl-4-aminophenol
- E4: 1-(2'-Hydroxyethyl)-2,5-diaminobenzol
- E5: p-Toluylendiamin
- E6: 1,10-Bis-(2,5-diaminophenyl)-1,4,7,10-tetraoxadecan
- K1: 3-Amino-2-chlor-6-methylphenol
- K2: 3-Trifluoroacetylamino-2-chlor-6-methylphenol
- K3: 5-Amino-4-chlor-2-methylphenol
- K4: 3-Amino-2,4-dichlorphenol
- K5: 2-Methylarnino-3-amino-6-methoxypyridin
- K6: 2,4-Diaminophenoxyethanol
Die Auswertung der Färbeergebnisse erfolgte visuell nach dem Taschenlexikon der Farben (12. Auflage, Musterschmidt-Verlag, Zürich, Göttingen 1975)

| Farbstoffkombination | Farbergebnis Luftoxidation | 1 % H₂O₂ | 9 % H₂O₂ |
|---|---|---|---|
| E 1 + K 3* | 5 B 5 | 6 B 5 | 6 B 5 |
| | grauorange | grauorange | grauorange |
| E 1 + K 4* | 5 F 5 | 6 F 3 | 5 F 5 |
| | nußbraun | negerbraun | nußbraun |
| E 1 + K S | 4 H 2 | 6 C 3 | 5 B 2 |
| | elfenbein | braunorange | marmorweiß |
| E 1 + K 1 + K 5 | S C 3 | 7 F 5 | 7 F 5 |
| | braunorange | dunkelbraun | dunkelbraun |
| E 1 + K 3 + K 6 * | 7 F 4 | 10 F 4 | 9 E 7 |
| | dunkelbraun | violettbraun | rotbraun |
| E 2 + K 3* | 6 A 2 | 7 A 4 | 5 A 4 |
| | orangeweiß | pfirsichrot | hellorange |
| E 2 + K 4* | 4 B 3 | 5 C 4 | 5 B 2 |
| | elfenbein | goldblond | marmorweiß |
| E 2 + K 5 | 5 C 5 | 5 F 3 | 5 F 3 |
| | topasgelb | nutria | nutria |
| E 2 + K 1 + K 2* | 5 B 3 | 7 A 4 | 7 A 5 |
| | grauorange | pfirsichrot | hellrot |
| E 2 + K 1 + K 5 | 4 B 3 | 7 E 3 | 7 E 2 |
| | elfenbein | graubraun | braungrau |
| E 1 + E 4 + K 3* | 6 A 2 | 10 C 4 | 12 D 6 |
| | orangeweiß | rotgold | graurubin |
| E 1 + E 6 + K 3* | 12 C 2 | 12 E 4 | 12 E 5 |
| | rotgrau | graurubin | graurubin |
| E 2 + E 4 + K 3* | 6 A 2 | 10 D 4 | 12 D 6 |
| | orangeweiß | dunkelblond | graurubin |
| E 2 + E 5 + K 3* | 6 A 2 | 11 D 4 | 12 E 7 |
| | orangeweiß | graurot | graurubin |
| E 3 + E 4 + K 3* | 8 B 2 | 10 C 4 | 12 D 5 |
| | rotgrau | rotgold | graurubin |
| E 1 + E 4 + K 1 + K 3* | 6 B 4 | 8 E 8 | 9 D 8 |
| | grauorange | rotbraun | granatbraun |
| E 1 + E 5 + K 1 + K 3* | 8 B 2 | 10 E 5 | 11 D 5 |
| | rotgrau | violettbraun | graurot |
| E 2 + E 4 + K 1 + K 3* | 8 B 2 | 11 C 4 | 12 E 5 |
| | rotgrau | mattrot | graurubin |
| E 2 + E 5 + K 1 + K 3* | 8 B 2 | 11 D 4 | 12 E 5 |
| | rotgrau | graurot | graurubin |

| | | | |
|---|---|---|---|
| *Beispiele sind nicht erfindungsgemäß | | | |

## Patentansprüche

1. Oxidationsfärbemittel zum Färben von Keratinfasern, insbesondere menschlichen Haaren, enthaltend in einem zum Färben geeigneten Medium mindestens ein p-Aminophenolderivat und mindestens ein Oxidationsmittel, ausgewählt aus Persulfaten, Chloriten sowie Wasserstoffperoxid oder dessen Anlagerungsprodukten an Harnstoff, Melamin sowie Natriumborat, **dadurch gekennzeichnet, dass** es mindestens eine Verbindung der Formel (II) oder eines deren physiologisch verträglichen Salze als Kupplerkomponente enthält 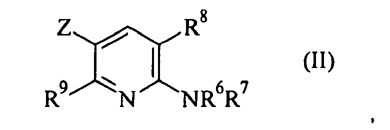 wobei R⁶ und R⁷ unabhängig voneinander stehen für Wasserstoff oder eine C₁₋₄₋ AlkylGruppe,
R⁸ und R⁹ stehen unabhängig voneinander für Wasserstoff, eine Aminogruppe, eine Hydroxygruppe oder eine C₁₋₄-Alkoxygruppe, und
Z steht für Wasserstoff oder ein Halogenatom, insbesondere ein Chloratom, wobei mindestens einer der Reste R⁶, R⁷, R⁹ oder Z von Wasserstoff verschieden ist,
mit der Maßgabe, dass das Mittel frei ist von 4-Amino-2-((diethylamino)-methyl)-phenol, wenn die Verbindung der Formel (II) 3-Amino-2-methylamino-6-methoxypyridin ist.

2. Mittel nach Anspruch 1, **dadurch gekennzeichnet, dass** das p-Aminophenol-Derivat ausgewählt ist aus Verbindungen der Formel (III) 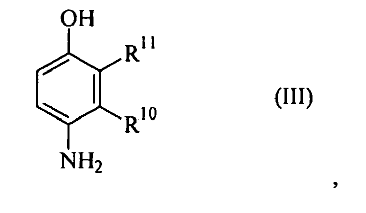 wobei
R¹⁰ und R¹¹ unabhängig voneinander stehen für Wasserstoff, eine C₁₋₄-Alkyl-Gruppe, eine C₁₋₄-Monohydroxyalkyl-Gruppe, eine C₂₋₄-Polyhydroxyalkyl-Gruppe, eine C₁₋₄-Alkoxy-C₁₋₄-Alkyl-Gruppe, eine C₁₋₄-Aminoalkyl-Gruppe, eine (C₁₋₄-Hydroxyalkyl)amino-Gruppe, eine ((C₁₋₄-Hydroxyalkyl)-amino)-C₁₋₄-Alkyl-Gruppe, eine ((Di-C₁₋₄-Alkyl)-amino)-C₁₋₄-AlkylGruppe, eine C₁₋₄-Hydroxyalkoxy-Gruppe, eine (2-Hydroxy-5-aminophenyl)-C₁₋₄-alkylGruppe, eine Carboxy-Gruppe oder ein Halogenatom.

3. Mittel nach einem der Ansprüche 1 oder 2, **dadurch gekennzeichnet, dass** die Verbindung der Formel (II) ausgewählt ist aus 3-Amino-2-methylamino-6-methoxypyridin, 2,3-Diamino-6-methoxypyridin, 2,6-Diaminopyridin und 2-Amino-3-hydroxy-5-chlorpyridin.

4. Mittel nach einem der Ansprüche 1 bis 3, **dadurch gekennzeichnet, dass** die Verbindung der Formel (III) ausgewählt ist aus Bis-(5-amino-2-hydroxyphenyl)-methan, 2-Aminomethyl-4-aminophenol, 4-Amino-2-((diethytamino)-methyl)-phenol, 2-(β-Hydroxyethylaminomethyl)-4-aminophenol, 3-Methyl-4-aminophenol und 5-Aminosalicylsäure.

5. Mittel nach einem der Ansprüche 1 bis 4, **dadurch gekennzeichnet, dass** weiterhin mindestens eine Verbindung der Formel (I) oder eines deren physiologisch verträglichen Salze als Kupplerkomponente 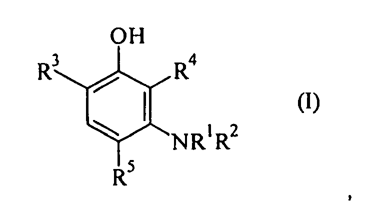 wobei R¹ und R² unabhängig voneinander stehen für Wasserstoff, eine Trifluoracetyl-Gruppe, eine C₁₋₄-Alkylgruppe, eine C₁₋₄-Monohydroxyalkylgruppe, eine C₂₋₄-Polyhydroxyalkylgruppe oder eine C₁₋₄-Aminoalkylgruppe,
R³, R⁴ und R⁵ stehen unabhängig voneinander für Wasserstoff, eine C₁₋₄-Alkyl-Gruppe, eine C₁₋₄-Alkoxy-Gruppe oder ein Halogenatom, insbesondere ein Chloratom, wobei mindestens einer der Reste R³, R⁴ und R⁵ für ein Halogenatom oder eine C₁₋₄-AlkoxyGruppe steht,
enthalten ist.

6. Mittel nach Anspruch 5, **dadurch gekennzeichnet, dass** die Verbindung der Formel (1) ausgewählt ist aus 5-Amino-4-chlor-2-methylphenol, 5-Amino-4-methoxy-2-methylphenol, 3-Trifluoroacetylamino-2-chlor-6-methylphenol, 3-Amino-2-chlor-6-methylphenol, 3-Amino-2,4-dichlorphenol, 3-Amino-6-chlorphenol, 3-Amino-6-bromphenol, 3-(β-Hydroxyethyl)-amino-6-chlorphenol und 3-(β-Aminoethyl)-amino-6-chlorphenol.

7. Mittel nach Anspruch 6, **dadurch gekennzeichnet, dass** die Verbindung der Formel (1) ausgewählt ist aus 5-Amino-4-chlor-2-methylphenol, 3-Trifluoroacetylamino-2-chlor-6-methylphenol, 3-Amino-2-chlor-6-methylphenol und 3-Amino-2,4-dichlorphenol.

8. Mittel nach einem der Ansprüche 1 bis 7, **dadurch gekennzeichnet, dass** es zur Nuancierung mindestens eine Kupplerkomponente, ausgewählt aus 3,5-Diamino-2,6-dimethoxypyridin, 1-Naphthol, 1,5-Dihydroxynaphthalin, 1,8-Dihydroxynaphthalin, 2,7-Dihydroxynaphthalin, m-Diaminobenzol, 2,4-Diaminophenoxyethanol, 1-Methoxy-2-amino-4-(2'-hydroxyethylamino)-benzol, 1-Amino-3-bis-(2'-hydroxyethyl)-aminobenzol und 1,3-Bis-(2,4-diaminophenoxy)-propan, 1,3-Bis-(2,4-diaminophenyl)-propan, 2,6-Bis-(2-hydroxyethylamino)-1-methylbenzol, 1-Hydroxy-3,4-methylendioxybenzol, 1-Amino-3,4-methylendioxybenzol und 1-(2'-Hydroxyethyl)-amino-3,4-methylendioxybenzol enthält.

9. Mittel nach einem der Ansprüche 1 bis 8, **dadurch gekennzeichnet, daß** es zur Nuancierung mindestens eine Entwicklerkomponente, ausgewählt aus der Gruppe, die aus p-Phenylendiamin, p-Toluylendiamin, N,N-Bis-(2-hydroxy-ethyl)-p-phenylendiamin, 1-(2'-Hydroxyethyl)-2,5-diaminobenzol, Bis-(4-aminophenyl)-amin, 1,10-Bis-(2,5-diaminophenyl)-1,4,7,10-tetraoxadecan, 2,6-Dichlor-4-aminophenol, 2,5-Diaminopyridin und 2-Dimethylamino-5-aminopyridin gebildet wird, enthält.

10. Mittel nach einem der Ansprüche 1 bis 9, **dadurch gekennzeichnet, dass** Entwicklerkomponenten in einer Menge von 0,005 bis 20 Gew.-%, vorzugsweise 0,1 bis 5 Gew.-%, und Kupplerkomponenten in einer Menge von 0,005 bis 20 Gew.-%, vorzugsweise 0,1 bis 5 Gew.%, jeweils bezogen auf das gesamte Oxidationsfärbemittel, enthalten sind.

11. Mittel nach einem der Ansprüche 1 bis 10, **dadurch gekennzeichnet, dass** mindestens ein direktziehender Farbstoff enthalten ist.

12. Verwendung von Mitteln nach einem der Ansprüche 1 bis 11 zum Färben keratinischer Fasern.

## Claims

1. Oxidation colorant for colouring keratin fibres, in particular human hair, comprising, in a medium suitable for the colouring, at least one p-aminophenol derivative and at least one oxidizing agent chosen from persulphates, chlorites, and hydrogen peroxide or its addition products onto urea, melamine, and sodium borate, **characterized in that** it comprises at least one compound of the formula (II) or one of its physiologically compatible salts as coupler component 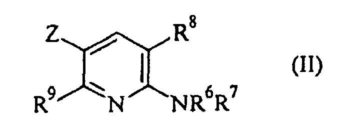 where R⁶ and R⁷, independently of one another, are hydrogen or a C₁₋₄-alkyl group,
R⁸ and R⁹, independently of one another, are hydrogen, an amino group, a hydroxy group or a C₁₋₄-alkoxy group, and
Z is hydrogen or a halogen atom, in particular a chlorine atom, where at least one of the radicals R⁶, R⁷, R⁹ or Z is different from hydrogen,
with the proviso that the agent is free from 4-amino-2-((diethylamino)methyl)phenol if the compound of the formula (II) is 3-amino-2-methylamino-6-methoxypyridine.

2. Agent according to Claim 1, **characterized in that** the p-aminophenol derivative is chosen from compounds of the formula (III) 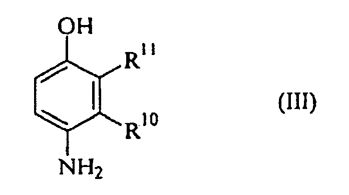 where
R¹⁰ and R¹¹, independently of one another, are hydrogen, a C₁₋₄-alkyl group, a C₁₋₄-monohydroxyalkyl group, a C₂₋₄-polyhydroxyalkyl group, a C₁₋₄-alkoxy-C₁₋₄-alkyl group, a C₁₋₄-aminoalkyl group, a (C₁₋₄-hydroxyalkyl)amino group, a ((C₁₋₄-hydroxyalkyl)amino)-C₁₋₄-alkyl group, a ((di-C₁₋₄-alkyl) amino) -C₁₋₄-alkyl group, a C₁₋₄-hydroxyalkoxy group, a (2-hydroxy-5-aminophenyl)-C₁₋₄-alkyl group, a carboxy group or a halogen atom.

3. Agent according to one of Claims 1 or 2, **characterized in that** the compound of the formula (II) is chosen from 3-amino-2-methylamino-6-methoxypyridine, 2,3-diamino-6-methoxypyridine, 2,6-diaminopyridine and 2-amino-3-hydroxy-5-chloropyridine.

4. Agent according to one of Claims 1 to 3, **characterized in that** the compound of the formula (III) is chosen from bis(5-amino-2-hydroxyphenyl)methane, 2-aminomethyl-4-aminophenol, 4-amino-2-((diethylamino)methyl)phenol, 2-(β-hydroxyethylaminomethyl)-4-aminophenol, 3-methyl-4-aminophenol and 5-aminosalicylic acid.

5. Agent according to one of Claims 1 to 4, **characterized in that** in addition at least one compound of the formula (I) or one of its physiologically compatible salts is present as coupler component 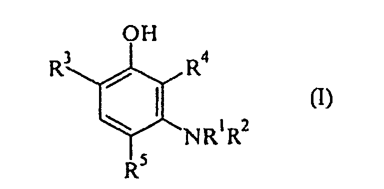 where R¹ and R², independently of one another, are hydrogen, a trifluoroacetyl group, a C₁₋₄-alkyl group, a C₁₋₄-monohydroxyalkyl group, a C₂₋₄-polyhydroxyalkyl group or a C₁₋₄-aminoalkyl group,
R³, R⁴ and R⁵, independently of one another, are hydrogen, a C₁₋₄-alkyl group, a C₁₋₄-alkoxy group or a halogen atom, in particular a chlorine atom, where at least one of the radicals R³, R⁴ and R⁵ is a halogen atom or a C₁₋₄-alkoxy group.

6. Agent according to Claim 5, **characterized in that** the compound of the formula (I) is chosen from 5-amino-4-chloro-2-methylphenol, 5-amino-4-methoxy-2-methylphenol, 3-trifluoroacetylamino-2-chloro-6-methylphenol, 3-amino-2-chloro-6-methylphenol, 3-amino-2,4-dichlorophenol, 3-amino-6-chlorophenol, 3-amino-6-bromophenol, 3-(β-hydroxyethyl)amino-6-chlorophenol and 3-((β-aminoethyl)amino-6-chlorophenol.

7. Agent according to Claim 6, **characterized in that** the compound of the formula (I) is chosen from 5-amino-4-chloro-2-methylphenol, 3-trifluoroacetylamino-2-chloro-6-methylphenol, 3-amino-2-chloro-6-methylphenol and 3-amino-2,4-dichlorophenol.

8. Agent according to one of Claims 1 to 7, **characterized in that** it comprises, for the nuancing, at least one coupler component chosen from 3,5-diamino-2,6-dimethoxypyridine, 1-naphthol, 1,5-dihydroxynaphthalene, 1,8-dihydroxynaphthalene, 2,7-dihydroxynaphthalene, m-diaminobenzene, 2,4-diaminophenoxyethanol, 1-methoxy-2-amino-4-(2'-hydroxyethylamino)-benzene, 1-amino-3-bis(2'-hydroxyethyl)aminobenzene and 1,3-bis(2,4-diaminophenoxy)propane, 1,3-bis(2,4-diaminophenyl)propane, 2,6-bis(2-hydroxyethylamino)-1-methylbenzene, 1-hydroxy-3,4-methylenedioxybenzene, 1-amino-3,4-methylenedioxybenzene and 1-(2'-hydroxyethyl) amino-3,4-methylenedioxybenzene.

9. Agent according to one of Claims 1 to 8, **characterized in that** it comprises, for the nuancing, at least one developer component chosen from the group which is formed from p-phenylenediamine, p-toluenediamine, N,N-bis(2-hydroxyethyl)-p-phenylenediamine, 1-(2'-hydroxyethyl)-2,5-diaminobenzene, bis(4-aminophenyl)amine, 1,10-bis(2,5-diaminophenyl)-1,4,7,10-tetraoxadecane, 2,6-dichloro-4-aminophenol, 2,5-diaminopyridine and 2-dimethylamino-5-aminopyridine.

10. Agent according to one of Claims 1 to 9, **characterized in that** developer components are present in an amount of from 0.005 to 20% by weight, preferably 0.1 to 5% by weight, and coupler components are present in an amount of from 0.005 to 20% by weight, preferably 0.1 to 5% by weight, in each case based on the total oxidation colorant.

11. Agent according to one of Claims 1 to 10, **characterized in that** at least one direct dye is present.

12. Use of agents according to one of Claims 1 to 11 for colouring keratin fibres.

## Revendications

1. Agent colorant d'oxydation destiné à la coloration de fibres kératiniques, en particulier de cheveux humains, contenant, dans un milieu approprié pour la coloration, au moins un dérivé de p-aminophénol et au moins un agent oxydant, choisi parmi les persulfates, les chlorites et le peroxyde d'hydrogène ou ses produits d'addition sur l'urée, la mélamine et le borate de sodium, **caractérisé en ce qu'**il contient au moins un composé de formule (II) ou un de ses sels physiologiquement acceptable en tant que composant de couplage 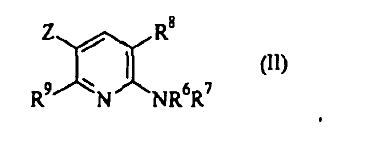 où
R⁶ et R⁷ représentent indépendamment un atome d'hydrogène ou un groupe alkyle en C₁₋₄,
R⁸ et R⁹ représentent indépendamment un atome d'hydrogène, un groupe amino, un groupe hydroxy ou un groupe alcoxy en C₁₋₄, et
Z représente un atome d'hydrogène ou un atome d'halogène, en particulier un atome de chlore, au moins l'un des groupes R⁶, R⁷, R⁹ ou Z étant autre que l'hydrogène, étant entendu que l'agent colorant est dépourvu de 4-amino-2-((diéthylamino)-méthyl)-phénol lorsque le composé de formule (II) est la 3-amino-2-méthylamino-6-méthoxypyridine.

2. Agent selon la revendication 1, **caractérisé en ce que** le dérivé de p-aminophénol est choisi parmi les composés de formule (III) 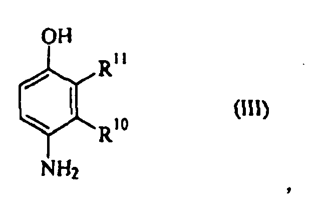 dans laquelle
R¹⁰ et R¹¹ représentent indépendamment un atome d'hydrogène, un groupe alkyle en C₁₋₄, un groupe monohydroxy(alkyle en C₁₋₄), un groupe polyhydroxy(alkyle en en C₂₋₄), un groupe (alcoxy en C₁₋₄)-(alkyle en C₁₋₄), un groupe amino(alkyle en C₁₋₄), un groupe (hydroxy(alkyle en C₁₋₄))amino, un groupe ((hydroxy(alkyle en C₁₋₄))amino)-(alkyle en C₁₋₄), un groupe (di-(alkyle en C₁₋₄))amino-(alkyle en C₁₋₄), un groupe hydroxy(alcoxy en C₁₋₄), un groupe (2-hydroxy-5-aminophényl)-(alkyle en C₁₋₄), un groupe carboxy ou un atome d'halogène.

3. Agent selon l'une quelconque des revendications 1 ou 2, **caractérisé en ce que** le composé de formule (II) est choisi parmi la 3-amino-2-méthylamino-6-méthoxypyridine, la 2,3-diamino-6-méthoxypyridine, la 2,6-diaminopyridine et la 2-amino-3-hydroxy-5-chloropyridine.

4. Agent selon l'une quelconque des revendications 1 à 3, **caractérisé en ce que** le composé de formule (III) est choisi parmi le bis-(5-amino-2-hydroxyphényl)-méthane, le 2-aminométhyl-4-aminophénol, le 4-amino-2-((diéthylamino)-méthyl)-phénol, le 2-(β-hydroxyéthylaminométhyl)-4-aminophénol, le 3-méthyl-4-aminophénol et l'acide 5-aminosalicylique.

5. Agent selon l'une quelconque des revendications 1 à 4, **caractérisé en outre en ce qu'**il contient au moins un composé de formule (I) ou un de ses sels physiologiquement acceptable en tant que composant de couplage 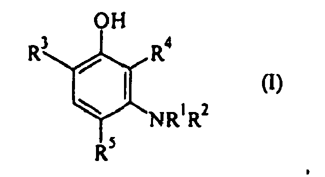 où R¹ et R² représentent indépendamment un atome d'hydrogène, un groupe trifluoroacétyle, un groupe alkyle en C₁₋₄, un groupe monohydroxy(alkyle en C₁₋₄), un groupe polyhydroxy(alkyle en C₂₋₄) ou un groupe amino(alkyle en C₁₋₄),
R³, R⁴ et R⁵ représentent indépendamment un atome d'hydrogène, un groupe alkyle en C₁₋₄, un groupe alcoxy en C₁₋₄ ou un atome d'halogène, en particulier un atome de chlore, au moins l'un des groupes R³, R⁴ et R⁵ représentant un atome d'halogène ou un groupe alcoxy en C₁₋₄.

6. Agent selon la revendication 5, **caractérisé en ce que** le composé de formule (I) est choisi parmi le 5-amino-4-chloro-2-méthylphénol, le 5-amino-4-méthoxy-2-méthylphénol, le 3-trifluoroacétylamino-2-chloro-6-méthylphénol, le 3-amino-2-chloro-6-méthylphénol, le 3-amino-2,4-dichlorophénol, le 3-amino-6-chlorophénol, le 3-amino-6-bromophénol, le 3-(β-hydroxyéthyl)-amino-6-chlorophénol et le 3-(β-aminoéthyl)-amino-6-chlorophénol.

7. Agent selon la revendication 6, **caractérisé en ce que** le composé de formule (I) est choisi parmi le 5-amino-4-chloro-2-méthylphénol, le 3-trifluoroacétylamino-2-chloro-6-méthylphénol, le 3-amino-2-chloro-6-méthylphénol et le 3-amino-2,4-dichlorophénol.

8. Agent selon l'une quelconque des revendications 1 à 7, **caractérisé en ce qu'**il contient, pour le nuançage, au moins un composant de couplage choisi parmi la 3,5-diamino-2,6-diméthoxypyridine, le 1-naphtol, le 1,5-dihydroxynaphtalène, le 1,8-dihydroxynaphtalène, le 2,7-dihydroxynaphtalène, le m-diaminobenzène, le 2,4-diaminophénoxyéthanol, le 1-méthoxy-2-amino-4-(2'-hydroxyéthylamino)-benzène, le 1-amino-3-bis-(2'-hydroxyéthyl)-aminobenzène et le 1,3-bis-(2,4-diaminophénoxy)-propane, le 1,3-bis-(2,4-diaminophényl)-propane, le 2,6-bis-(2-hydroxyéthylamino)-1-méthylbenzène, le 1-hydroxy-3,4-méthylènedioxybenzène, le 1-amino-3,4-méthylènedioxybenzène et le 1-(2'-hydroxyéthyl)-amino-3,4-méthylènedioxybenzène.

9. Agent selon l'une quelconque des revendications 1 à 8, **caractérisé en ce qu'**il contient, pour le nuançage, au moins un composant développeur, choisi parmi la p-phénylènediamine, la p-toluylènediamine, la N,N-bis-(2-hydroxyéthyl)-p-phénylènediamine, le 1-(2'-hydroxyéthyl)-2,5-diaminobenzène, la bis-(4-aminophényl)-amine, le 1,10-bis-(2,5-diaminophényl)-1,4,7,10-tétraoxadécane, le 2,6-dichloro-4-aminophénol, la 2,5-diaminopyridine et la 2-diméthylamino-5-aminopyridine.

10. Agent selon l'une quelconque des revendications 1 à 9, **caractérisé en ce que** le composant développeur est contenu dans une proportion de 0,005 à 20 % en poids, de préférence de 0,1 à 5 % en poids et le composant de couplage est contenu dans une proportion de 0,005 à 20 % en poids, de préférence de 0,1 à 5 % en poids, toujours par rapport à la quantité totale de l'agent colorant d'oxydation.

11. Agent selon l'une quelconque des revendications 1 à 10, **caractérisé en ce qu'**il contient au moins un colorant montant directement.

12. Utilisation d'un agent selon l'une quelconque des revendications 1 à 11 pour la coloration de fibres kératiniques.
